# EUROPEAN PATENT APPLICATION

(11) **EP 4 516 732 A1**
(43) Date of publication of application: **05.03.2025**
(21) Application number: 23796225.3
(22) Date of filing: 20.04.2023
(51) Int. Cl.: C01B 21/064, A61K 8/19, A61Q 1/02

(54) **HEXAGONAL BORON NITRIDE POWDER AND METHOD FOR PRODUCING SAME**

(30) Priority: 26.04.2022 JP 2022072265
(71) Applicant: TOKUYAMA CORPORATION, Yamaguchi 745-8648 (JP)
(72) Inventor: ABURATANI, Makoto, Shunan-shi, Yamaguchi 745-8648 (JP)
(74) Representative: Kraus & Lederer PartGmbB
(86) International application number: PCT/JP2023/015724
(87) International publication number: WO 2023/210483

(57) **Abstract**

There is provided a hexagonal boron nitride powder, having: an average long diameter (L50) of 3 to 20 µm, an average thickness (T50) of 0.3 to 2 µm, a (L90-L10)/L50 of 2.0 or less, a (T90-T10)/T50 of 2.0 or less, and an average aspect ratio of 10 to 30; a dynamic friction coefficient (MIU) of 0.50 or less, and a deviation of the dynamic friction coefficient (MMD) of 0.0050 or less; and a specular reflection intensity of 80 or more as measured by a goniophotometer under a condition of an angle of incidence of 60°C.

## Description

### Technical Field:

The present invention relates to a hexagonal boron nitride powder (hereinafter, may be abbreviated as a "boron nitride powder" or a "h-BN powder".) More specifically, the present invention relates to a hexagonal boron nitride powder suitable for use in cosmetics and a method for producing the same.

### Background Art:

Inorganic materials such as talc, mica and kaolin, and resin materials such as nylon powder and polyethylene powder have been used as extender pigments for cosmetics. Extender pigments, which serve as a base for dispersing various components contained in cosmetic such as a color pigment, greatly affect glossiness and usability, such as spreadability (i.e., the property of ensuring a smooth application on the skin surface) and lasting (i.e., the property of long lasting on the skin).

However, these materials are not always satisfactory in terms of usability and stability. For example, inorganic materials such as talc, mica and kaolin allegedly cause a change in odor, as their catalytic activity can contribute to the degradation of perfume and oil. Resin materials such as nylon powder and polyethylene powder are chemically stable but are poor in formability.

Hence, hexagonal boron nitride, which has a flattened shape and superior lubricity to other materials, is being used as a white extender pigment for cosmetics.

Patent Document 1 describes a boron nitride powder containing plate-like aggregates of stacked primary particles, each having a flattened shape with predetermined average long diameter and thickness. This powder is obtained by reacting boric acid, urea and boron carbide so that oxygen and carbon are contained at a predetermined ratio.

Patent Document 2 describes a hexagonal boron nitride powder having predetermined average particle diameter, maximum particle diameter, and specific surface area, as well as predetermined graphitization index, mean friction coefficient, and deviation of the mean friction coefficient. This powder can ensure improved smoothness and reduced roughness. Patent Document 2 describes a method for producing the hexagonal boron nitride powder in accordance with specified first and second firing conditions.

Patent Document 3 proposes a boron nitride powder having predetermined water permeation rate and oil absorption, based on the findings that the oil absorption of a component powder of a cosmetic is closely related to the property of long "lasting" on the skin. The boron nitride powder of Patent Document 3 is produced by heating boric acid, urea and boron carbide in two stages.

Patent Document 4 proposes a boron nitride powder defined such that its elution amount of boron, average particle diameter, and content of particles with a specific particle diameter are not more than predetermined values. This powder has excellent "moist" and "slimy" textures while maintaining appropriate smoothness; thus, it is suitable for use in cosmetics. Patent Document 4 describes the mean friction coefficient and deviation of the mean friction coefficient of this boron nitride powder. Patent Document 4 also describes a method for producing a boron nitride powder, which includes two-stage firing, pulverization and washing.

Patent Document 5 proposes a hexagonal boron nitride powder having predetermined specific surface area, long diameter of primary particles, and aspect ratio. This powder can be used to achieve a resin sheet with high thermal conductivity and high dielectric strength. Patent Document 5 describes a method for producing a hexagonal boron nitride powder by mixing boron oxide, a nitrogen-containing organic compound, and lithium carbonate at a predetermined weight ratio and heating the mixture.

Patent Document 6 describes a method for producing a hexagonal boron nitride powder with a low aspect ratio by disintegrating a raw material powder without substantially pulverizing primary particles. The raw material powder contains hexagonal boron nitride particles with an aspect ratio in a predetermined range and hexagonal boron nitride particles with an aspect ratio higher than a predetermined value.

Patent Document 7 teaches "by increasing the proportion of boron nitride particles in a boron nitride powder that have a bent structure at a specific angle, it is possible to improve the glitter property of the boron nitride powder when used alone and the glitter property when used in combination with a glitter pigment". A goniophotometer is used for the measurement of particles having bent structure at certain angles in Patent Document 7.

Non-Patent Document 1, which is directed to delamination behavior of two-dimensional nanofillers by wet-type jet milling, teaches the use of hexagonal boron nitride and graphite as two-dimensional nanofillers.

However, these conventional boron nitride powders are not entirely satisfactory in terms of achieving both glossiness and a high level of usability desired by cosmetic users.

### Prior Art Documents:

### Patent Documents:

Patent Document 1: JP-A-2012-176910
Patent Document 2: JP-A-2018-165241
Patent Document 3: WO2014/049956
Patent Document 4: WO2019/172440
Patent Document 5: WO2020/179662
Patent Document 6: WO2021/085223
Patent Document 7: JP-B1-6625308

### Non-Patent Documents:

Non-Patent Document 1: Effect of Medium Viscosity on Delamination Behavior of Two-dimensional Nanofillers by Wet-type Jet Milling, Yusuke Imai and Yuji Hotta, J. Soc. Powder Technol., Japan, 54, 442-447 (2017)

### Summary of the Invention:

### Problems to be solved by the Invention:

An object of the present invention is to provide a cosmetic containing a hexagonal boron nitride powder that ensures good smoothness, a moist texture (fits well with the skin), reduced roughness, and excellent glossiness, and a hexagonal boron nitride powder for use in the preparation of the cosmetic. Another object of the present invention is to provide a method for producing the boron nitride powder.

### Means for Solving the Problems:

The present inventor has found that: using a boron nitride powder pulverized by a wet-type jet mill as a seed crystal is a key to produce a hexagonal boron nitride powder with uniform long diameter and thickness by the reduction nitridation method which involves heating a raw material mixture in a nitrogen atmosphere; and a cosmetic prepared using the thus-produced boron nitride powder with uniform long diameter and thickness has good smoothness, a moist texture (fits well with the skin), reduced roughness, and excellent glossiness. Based on these findings, the present invention has been completed.

The present invention encompasses the following inventions.
1. A hexagonal boron nitride powder, having:
   an average long diameter (L50) of 3 to 20 µm, an average thickness (T50) of 0.3 to 2 µm, a (L90-L10)/L50 of 2.0 or less, a (T90-T10)/T50 of 2.0 or less, and an average aspect ratio of 10 to 30;
   a dynamic friction coefficient (MIU) of 0.50 or less, and a deviation of the dynamic friction coefficient (MMD) of 0.0050 or less; and
   a specular reflection intensity of 80 or more as measured by a goniophotometer under a condition of an angle of incidence of 60°.
2. The hexagonal boron nitride powder according to 1 above, wherein the (L90-L10)/L50 is 1.8 or less.
3. The hexagonal boron nitride powder according to 1 above, for use in a cosmetic.
4. A cosmetic including the hexagonal boron nitride powder according to 1 above.
5. A method for producing a hexagonal boron nitride powder, including reducing and nitriding a raw material mixture by heating in a nitrogen atmosphere, wherein
   the raw material mixture contains an oxygen-containing boron compound, an oxygen-containing alkaline earth metal compound, a carbon source, and a seed crystal, and
   the seed crystal is a boron nitride powder with a specific surface area of 10 m²/g or more obtained by subjecting a raw material boron nitride powder with a graphitization index of 1.7 or less to wet-type jet milling.
6. The method for producing a hexagonal boron nitride powder according to 5 above, wherein the raw material boron nitride powder to be subjected to wet-type jet milling is in the form of a slurry with a density of 20 to 100 g/L.
7. The method for producing a hexagonal boron nitride powder according to 5 above, wherein the raw material mixture has:
   an atomic ratio (B/C ratio) of boron atoms (B) in the oxygen-containing boron compound to carbon atoms (C) in the carbon source of 0.70 to 2.00;
   a molar ratio (AO/B₂O₃ ratio) of the oxygen-containing alkaline earth metal compound (AO; A is an alkaline earth metal) to the oxygen-containing boron compound (B₂O₃) of 0.01 to 1.0 in terms of oxide; and
   an atomic ratio (B^{S}/C ratio) of boron atoms (B^{S}) in the boron nitride powder as the seed crystal to the carbon atoms (C) in the carbon source of 0.01 to 13.
8. The method for producing a hexagonal boron nitride powder according to 5 above, wherein the raw material mixture is reduced and nitrided by heating at a temperature of 1700°C to 2100°C.

### Effect of the Invention:

The hexagonal boron nitride powder of the present invention has a uniform long diameter with a sharp long diameter distribution and a uniform thickness with a sharp thickness distribution. Thus, the cosmetic containing this hexagonal boron nitride powder has good smoothness, a moist texture (fits well with the skin), reduced roughness, and excellent glossiness. The method for producing a boron nitride powder according to the present invention is capable of producing a hexagonal boron nitride powder having a uniform long diameter with a sharp long diameter distribution and a uniform thickness with a sharp thickness distribution.

### Brief Description of the Drawings:

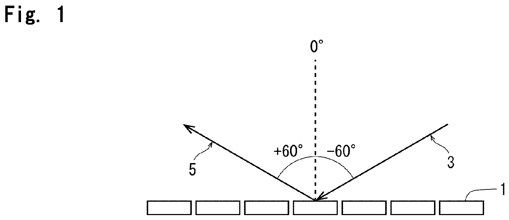

[Figure 1]: a schematic diagram for explaining the measurement of the specular reflection intensity of a hexagonal boron nitride powder of the present invention that is performed using a goniophotometer under the condition that the angle of incidence is 60°.

### Mode for Carrying out the Invention:

### <Hexagonal boron nitride powder>

### [Average long diameter, average thickness and average aspect ratio]

A boron nitride powder is dispersed in epoxy resin or the like, followed by curing. The resultant cured product is milled on the side, and the milled surface is imaged by an SEM. The long side and short side of a boron nitride particle observed in the SEM image are regarded as the long diameter and thickness, respectively, of the particle. When the boron nitride particles are arranged in ascending order of long diameter and thickness, the values at the area-based 50% cumulative frequency are determined as the average long diameter (L50) and average thickness (T50), respectively, of the boron nitride particles.

The average long diameter (L50) of the boron nitride of the present invention is 3 to 20 µm, preferably 4 to 15 µm, and more preferably 5 to 12 µm. The average thickness (T50) of the boron nitride particle is 0.3 to 2 µm, preferably 0.4 to 1.2 µm.

If the average long diameter (L50) is less than 3 µm, the boron nitride powder will have poor smoothness. If the average long diameter (L50) is more than 20 µm, the powder will be too smooth to impart any "moist" and "slimy" textures to a cosmetic and will be too glittery in appearance; thus, such a powder is not suitable as a cosmetic raw material. On the other hand, if the average thickness (T50) is less than 0.3 µm, the powder will provide poor coverage of wrinkles and dark spots. If the average thickness (T50) is more than 2 µm, the powder will be less transparent, providing makeup with an unnatural finish.

The content of coarse particles that are more than 2.5 times as large as the average long diameter (L50) is 12 area% or less, preferably 9 area% or less, and more preferably 6 area% or less. A smaller content of the coarse particles results in a "moist" texture and suppressed roughness.

The average aspect ratio is the value (= L50/T50) obtained by dividing the average long diameter (L50) of the particle by the average thickness (T50). The average aspect ratio is 10 to 30, preferably 12 to 28, and more preferably 14 to 25. The particle with an average aspect ratio of more than 10 achieves dramatically excellent spreadability, smoothness and adhesion. Meanwhile, a particle with an average aspect ratio of more than 30 is hard to produce.

The boron nitride powder of the present invention has a (L90-L10)/L50 of 2.0 or less, preferably 1.9 or less, and more preferably 1.8 or less. A smaller (L90-L10)/L50 indicates a more uniform long diameter with a sharper long diameter distribution, leading to better smoothness and a more "moist" texture of cosmetics. The boron nitride powder with a sharp long diameter distribution contains a high proportion of particles having an average long diameter (L50) close to the preferred range. Since such a boron nitride powder has suppressed roughness as a whole, it can impart higher smoothness and a more "moist" texture to cosmetics. In the present invention, the L10, L50 and L90 of the boron nitride powder refer to the area-based cumulative 10% value (L10), cumulative 50% value (L50), and cumulative 90% value (L90), respectively, of the boron nitride particles as arranged in ascending order of long diameter.

The boron nitride powder of the present invention has a (T90-T10)/T50 of 2.0 or less, preferably 1.9 or less, and more preferably 1.6 or less. A smaller (T90-T10)/T50 indicates a more uniform thickness with a sharper thickness distribution, which is important in imparting glossiness in the present invention. The boron nitride powder with a sharp thickness distribution is less uneven and almost flat on the surface. Thus, light incident on such a boron nitride powder is less likely to be diffusely reflected in various directions. Instead, a larger amount of light is regularly reflected in a certain direction, which leads to increased glossiness. Further, when not only the thickness distribution but also the long diameter distribution is sharp, influence by eneven on the surface of the powder is much less, resulting in further increased glossiness. In the present invention, the T10, T50 and T90 of the boron nitride powder refer to the area-based cumulative 10% value (T10), cumulative 50% value (T50), and cumulative 90% value (T90), respectively, of the boron nitride particles as arranged in ascending order of thickness.

### [MIU and MMD]

MIU (friction coefficient) is a parameter for quantifying smoothness. A lower MIU value indicates better smoothness. Meanwhile, MMD (deviation of friction coefficient) is a parameter for quantifying roughness. A lower MMD value indicates lower roughness.

The dynamic friction coefficient (MIU) of the boron nitride powder of the present invention is 0.50 or less, preferably 0.42 or less, and more preferably 0.40 or less. The boron nitride powder with a MIU of 0.50 or less can impart improved smoothness to a prepared cosmetic.

The deviation of the dynamic friction coefficient (MMD) of the boron nitride powder of the present invention is 0.0050 or less, preferably 0.0045 or less, and more preferably 0.0040 or less. The boron nitride powder with a MMD of 0.0050 or less can suppress roughness and impart improved usability to a prepared cosmetic.

The "MIU (friction coefficient)" and the "MMD (deviation of friction coefficient)" as used herein are dimensionless numbers measured by a friction tester (manufactured by KATO TECH CO., LTD. under the trade name of KES-SE). More specifically, the boron nitride powder is placed on artificial leather (manufactured by Idemitsu Technofine, Co., Ltd. under the trade name of SUPPLALE PBZ13001 BK) that simulates the skin, such that the powder is spread thinly in an amount of 0.5 mg/cm². Then, a (10-mm square silicon) sensor portion of the friction tester is applied onto the powder, thereby measuring the MIU and the MMD. The measurement conditions of the friction tester are set as follows: the sensitivity is H; the test table moving speed is 1 mm/second; and the static load is 25 gf. The measurement is performed once. The operation of preparing the specimen (by applying the boron nitride powder onto the artificial leather), followed by the measurement by the friction tester, is repeated five times. The average of the five measurements is determined as the MIU/MMD of the boron nitride powder.

### [Specular reflection intensity]

The specular reflection intensity of the boron nitride powder of the present invention indicates the degree of specular reflection as measured by a goniophotometer. A higher intensity value indicates a more intense specular reflection, which contributes to increased glossiness of the boron nitride powder. The specular reflection intensity of the boron nitride powder of the present invention as measured under the condition that the angle of incidence is 60° is 80 or more, preferably 82 or more, and more preferably 88 or more. If the specular reflection intensity is less than 80, the powder will have poor glossiness and, thus, is not suitable as a cosmetic raw material.

Figure 1 is a schematic diagram for explaining the measurement of the specular reflection intensity of a hexagonal boron nitride powder 1 of the present invention that is performed under the condition that the angle of incidence is 60°.

In the present invention, light with an angle of incidence of 60° refers to light (incident light 3) that is incident at an angle of -60° with respect to the line (which is assumed to have an angle of 0°) perpendicular to the surface (that is formed of the hexagonal boron nitride powder 1) exposed to the light. Light specularly reflected from the hexagonal boron nitride powder 1 in the same plane is reflected light 5. In Figure 1, peak reflected light observed at an angle of +60° is shown representatively as the reflected light 5. In the present invention, the specular reflection intensity is a value obtained by measuring the peak reflected light 5 by a goniophotometer.

### <Cosmetic>

The above-described hexagonal boron nitride powder can be used in cosmetics. The present invention encompasses a cosmetic containing the hexagonal boron nitride powder. The hexagonal boron nitride powder of the present invention, when used in a cosmetic, serves as a pigment for the cosmetic, allowing the prepared cosmetic to have good smoothness, a moist texture (fit well with the skin), reduced roughness, and excellent glossiness.

Examples of the cosmetic include foundation (such as powder foundation, liquid foundation, and cream foundation), face powder, point makeup, eye shadow, eyeliner, nail polish, lipstick, blusher, and mascara. Among them, the cosmetic of the present disclosure is particularly well adapted to powder foundation and face powder.

The content of the boron nitride powder in the cosmetic is, for example, preferably 1 to 70 mass%, more preferably 3 to 40 mass%, and still more preferably 8 to 30 mass%, of the total amount of the cosmetic.

The cosmetic may contain other components, including: pigments such as colcothar, yellow iron oxide, black iron oxide, titanium oxide, silica, aluminum hydroxide, and mica; esters such as diisostearyl malate and glyceryl tri(2-ethylhexanoate); and oils such as petrolatum. Further examples include talc, silicone powder, urethane powder, methyl paraben, and sodium dehydroacetate.

<Raw material boron nitride powder>

A raw material boron nitride powder is a boron nitride powder produced in advance as a raw material for a seed crystal (S). Specifically, the raw material boron nitride powder is cleaved by wet-type jet milling, thereby obtaining the seed crystal (S).

In the present invention, the raw material boron nitride powder is characterized by its high crystallinity. The degree of crystallinity can be found by measuring the graphitization index (GI) of the boron nitride powder. A lower graphitization index indicates higher crystallinity. The graphitization index of the raw material boron nitride powder is 1.7 or less, preferably 1.6 or less. When the raw material boron nitride powder has high crystallinity, the crystal can be easily cleaved along the (001) plane during wet-type jet milling, resulting in a seed crystal boron nitride powder with a small thickness. Such a seed crystal can be used to obtain a boron nitride powder having a high aspect and a sharp thickness distribution. The graphitization index of the boron nitride powder can be measured by a known method such as X-ray diffraction.

The raw material boron nitride powder preferably has a L50 of 10.0 µm or less, more preferably 5.0 µm or less. The raw material boron nitride powder preferably has a T50 of 1.5 µm or less, more preferably 0.8 µm or less. If the L50 and the T50 are too large, jet milling will take a long time to obtain a desired seed crystal, resulting in reduced production efficiency. Meanwhile, when the L50 and the T50 are small, it is difficult for the raw material boron nitride powder to have a graphitization index of 1.7 or less. Considering that such ranges are difficult to measure, the raw material boron nitride powder may be controlled by its specific surface area, which correlates with the L50 and the T50. The specific surface area of the raw material boron nitride powder tends to be larger when the L50 and the T50 are smaller, and is preferably 15 m²/g or less, more preferably 10 m²/g or less, and still more preferably 5 m²/g or less.

The raw material boron nitride powder preferably has an aspect ratio of 6 to 30, more preferably 7 to 25. If the aspect ratio is too small, jet milling will take a long time to obtain a desired seed crystal, resulting in reduced production efficiency. A raw material boron nitride powder with an aspect ratio of larger than 30 is hard to produce.

Any raw material boron nitride powder is applicable without limitation, as long as it has a graphitization index of 1.7 or less. Examples of the raw material boron nitride powder include those produced by known methods such as the melamine method and the reduction nitridation method. A raw material boron nitride powder produced by the melamine method is more preferred for easy control of uniform long diameter and thickness. Alternatively, a commercially available boron nitride powder may also be used.

### <Seed crystal (S)>

The seed crystal (S) is a hexagonal boron nitride powder obtained by cleaving, preferably followed by pulverizing, the raw material boron nitride powder by wet-type jet milling. As described above, the high crystallinity of the raw material boron nitride powder allows the seed crystal (S) to have a high aspect ratio and a sharp thickness distribution.

Since the seed crystal (S) is obtained by cleaving the raw material boron nitride powder, its particles are so small that the long diameter and the thickness are difficult to measure. As such, the specific surface area, which correlates with the L50 and the T50, is measured to help indirectly control the long diameter and the thickness. The specific surface area of the seed crystal (S) is 10 m²/g or more, preferably 11 m²/g or more, and still more preferably 12 m²/g or more. The seed crystal (S) with a larger specific surface area contains particles that are more thinly exfoliated or more finely pulverized, and tends to have a smaller (L90-L10)/L50 or (T90-T10)/T50. Such a seed crystal (S) may be used as part of the raw material to produce a hexagonal boron nitride powder by the reduction nitridation method, which also allows the resultant hexagonal boron nitride powder to have a sharp long diameter distribution and a sharp thickness distribution.

The ratio (A₂/A₁) between the specific surface area (A₁) of the raw material boron nitride powder and the specific surface area (A₂) of the seed crystal (S) is preferably 1.6 to 30, more preferably 1.8 to 20. If the A₂/A₁ is higher than 30, the seed crystal (S) will be difficult to handle because of excessive cleavage (or pulverization) by wet-type jet milling. If the A₂/A₁ is lower than 1.6, the desired seed crystal (S) may not be produced because of insufficient cleavage (or pulverization) by wet-type jet milling.

### <Method for producing hexagonal boron nitride powder>

A method for producing the hexagonal boron nitride powder according to the present invention is characterized in that a raw material mixture (S) is reduced and nitrided by heating in a nitrogen atmosphere in the presence of the seed crystal (S) obtained by cleaving, preferably followed by pulverizing, the raw material boron nitride powder by wet-type jet milling. This production method will be described below.

### <Method for producing raw material boron nitride powder>

First, a description will be given of a method for producing the raw material boron nitride powder for use as a raw material. The raw material boron nitride powder is produced by the melamine method or the reduction nitridation method. The respective production methods will be described below.

### <Production of raw material boron nitride powder by melamine method>

The melamine method involves heating a raw material mixture (M) containing an oxygen-containing boron compound and a nitrogen-containing organic compound in a nitrogen atmosphere.

### <Oxygen-containing boron compound>

Examples of the oxygen-containing boron compound include boric acid, boric anhydride (boron oxide), metaboric acid, perboric acid, sub-boric acid, sodium tetraborate, and sodium perborate.

### <Nitrogen-containing organic compound>

Examples of the nitrogen-containing organic compound include melamine, ammeline, cyandiamide, and urea.

### <Oxygen-containing alkaline earth metal compound>

The raw material mixture (M) may also contain an oxygen-containing alkaline earth metal compound. Examples of the oxygen-containing alkaline earth metal compound can include magnesium carbonate (MgCO₃), calcium carbonate (CaCO₃), and calcium oxide (CaO). Two or more of these compounds may be used in combination.

### <Composition ratio of raw material mixture (M) in melamine method>

The raw material mixture (M) used to produce the raw material boron nitride powder preferably has a boron/nitride (B/N) element ratio of 0.2 to 1, more preferably 0.25 to 0.5.

### <Heating step>

The heating temperature is preferably 500°C to 1200°C, more preferably 600°C to 1100°C, and still more preferably 650°C to 1000°C. A higher heating temperature contributes to higher crystallinity of the resultant raw material boron nitride powder.

The heating time is preferably 5 to 20 hours, more preferably 6 to 15 hours. A longer heating time contributes to higher crystallinity of the resultant raw material boron nitride powder.

The furnace atmosphere preferably includes a non-oxidizing gas, such as nitrogen, ammonia, hydrogen, helium, or argon. For reasons of availability and affordability, nitrogen is preferred.

A container for the raw material mixture (M) may be prepared, such as a carbon container, a boron nitride-coated carbon container, or a boron nitride sintered body container, and the container filled with the raw material mixture (M) may be placed in a furnace.

To enhance the efficiency of subsequent wet-type jet milling, it is preferable to reduce aggregated particles of the raw material boron nitride powder in advance. Aggregated particles can be reduced by disintegrating or classifying the reaction product.

### <Disintegration step>

In the disintegration step, the reaction product is preferably disintegrated with such force as not to pulverize the boron nitride particles, suitably by a stone disintegrator, a roll crusher or the like. If a dry-type jet mill is used, the boron nitride particles may be pulverized with excessive force applied.

### <Washing step>

The reaction product obtained by the melamine method contains impurities such as unreacted raw materials (B₂O₃, CaO). Such impurities and the like are removed by washing the reaction product with acid, which allows for wet-type jet milling with increased efficiency.

The method of acid washing is not particularly limited, and any known method is applicable without limitation. For example, the reaction product obtained by the melamine method is manually placed in a container, to which dilute hydrochloric acid (5 to 20 mass% HCl) is added in an amount 3 to 10 times that of the reaction product, followed by stirring for 1 to 20 hours.

After the acid washing, the resultant product is washed with pure water for the removal of the acid. The pure water available for use in the washing is water with an electrical conductivity of 1 mS/m or less for the avoidance of secondary contamination by impurities. The washing is performed as follows: After filtration of the acid used for the acid washing, pure water is added in the same amount as the acid used, followed by another filtration. This operation of pure water washing and filtration is repeated until the filtrate is neutralized.

After the washing, the resultant product may be subjected to wet-type jet milling without being dried, or alternatively may be dried and then slurried for use in wet-type jet milling.

### <Drying step>

The raw material boron nitride powder can be dried, for example, using a shelf-type dryer, a fluidized-bed dryer, a spray dryer, a rotary dryer, a belt dryer, a vacuum dryer, a vibration dryer, or a combination thereof. The atmosphere temperature in the dryer is preferably 50°C to 300°C, more preferably 100°C to 250°C. The drying time is not particularly specified, but is usually recommended to be 1 to 48 hours at the aforementioned temperature, as it is preferable for the powder to be dried to a moisture content as close to 0% as possible. Each of the washing and the drying may be performed once or repeated a plurality of times by the same method or in combination with another method.

### <Classification step>

The method of classification is not particularly limited, and any known method is applicable without limitation. Specific examples include a vibrating sieving machine, a wet sieving machine, a wind classifier, a cyclone, and a liquid cyclone. For reasons of ease of operation, a sieving machine such as a vibrating sieving machine or a wet sieving machine is preferably used. The sieve opening is not particularly limited and may be determined according to the intended use, but is preferably 25 to 90 µm, particularly preferably 25 to 45 µm. The sieve opening in this range helps to facilitate efficient wet-type jet milling. If the sieve opening is less than 25 µm, the classification step will take a long time. The classification may be dry classification that precedes both the washing and the drying, wet classification that follows the washing and precedes the drying, or dry classification that follows both the washing and the drying. Considering that the washing for removing impurities may serve to facilitate the classification, the classification preferably follows the washing and precedes the drying, or follows both the washing and the drying.

The raw material boron nitride powder produced by the melamine method has relatively uniform long diameter and thickness. This is presumably because the reaction temperature (i.e., the temperature of formation of boron nitride) in the melamine method is as low as 500°C to 1200°C, resulting in a boron nitride powder with relatively uniform long diameter and thickness. In contrast, the reduction nitridation method is presumably less likely to produce a raw material boron nitride powder with relatively uniform long diameter and thickness, because the reaction temperature is as high as 1400°C or more, at which the formation of boron nitride proceeds simultaneously with crystal growth. Using the raw material boron nitride with uniform long diameter and thickness makes it easier to obtain a boron nitride powder (seed crystal (S)) with uniform long diameter and thickness after wet-type jet milling, as will be described later. For this reason, the raw material boron nitride powder is preferably produced by the melamine method.

### <Production of raw material boron nitride powder by reduction nitridation method>

The reduction nitridation method involves heating a raw material mixture (R) containing an oxygen-containing boron compound, an oxygen-containing alkaline earth metal compound, and a carbon source in a nitrogen atmosphere, thereby obtaining a raw material boron nitride powder.

### <Oxygen-containing boron compound>

Examples of the oxygen-containing boron oxide can include diboron trioxide (boron oxide), diboron dioxide, tetraboron trioxide, tetraboron pentoxide, borax, and anhydrous borax. Among them, diboron trioxide (B₂O₃) is preferred. It is industrially beneficial to use diboron trioxide as the boron oxide, as it is less expensive. Two or more of the oxides may be used in combination as the boron oxide.

### <Oxygen-containing alkaline earth metal compound>

Examples of the oxygen-containing alkaline earth metal compound can include oxides and carbonates of an alkaline earth metal such as calcium or magnesium. Specific examples can include magnesium oxide, calcium oxide, magnesium carbonate, calcium carbonate, magnesium hydrogencarbonate, calcium hydrogencarbonate, magnesium hydroxide, calcium hydroxide, magnesium nitrate, calcium nitrate, magnesium sulfate, calcium sulfate, magnesium phosphate, calcium phosphate, magnesium oxalate, and calcium oxalate. When a carbonate, such as magnesium carbonate or calcium carbonate, is used as the oxygen-containing alkaline earth metal compound, carbon dioxide gas is generated in the heating step, making a layer of the raw material mixture porous. This allows nitrogen gas to be easily infiltrated into the layer of the raw material mixture, helping the reduction nitridation reaction to proceed efficiently. Two or more of the compounds may be used in combination as the oxygen-containing alkaline earth metal compound.

### <Carbon source>

Examples of the carbon source include: amorphous carbon such as carbon black, activated carbon, and carbon fiber; crystalline carbon such as diamond, graphite, and nanocarbon; and pyrolytic carbon obtained by thermal decomposition of a monomer or a polymer. Usually, carbon black is used, which is less expensive.

<Composition ratio of raw material mixture (R) in reduction nitridation step>

The raw material mixture (R) in the reduction nitridation method for producing the raw material boron nitride powder preferably has an atomic ratio (B/C ratio) of boron atoms (B) in the oxygen-containing boron compound to carbon atoms (C) in the carbon source of 0.70 to 2.00, more preferably 0.75 to 1.95. If the B/C ratio is smaller than this range, unreacted carbon unfavorably remains in the raw material boron nitride powder as a black foreign material. If the B/C ratio is larger than the range, unreacted B₂O₃ accounts for an excessive proportion of the raw material boron nitride powder, which causes a significant reduction in the yield of boron nitride for the amount of the filling raw material, resulting in lower productivity.

The raw material mixture preferably contains the oxygen-containing alkaline earth metal compound (AO; A is an alkaline earth metal) and the oxygen-containing boron compound at a molar ratio (AO/B₂O₃) of 0.01 to 1.0, more preferably 0.03 to 0.90, and still more preferably 0.05 to 0.85, in terms of oxide. The oxygen-containing alkaline earth metal compound, which serves as a growth aid for boron nitride particles, is blended so that the AO/B₂O₃ ratio falls within this range, resulting in a raw material boron nitride powder with a graphitization index of 1.7 or less.

### <Heating step>

The reduction nitridation method involves heating the raw material mixture (R) under a nitrogen stream. The boron nitride (BN) powder may be produced from boron oxide (B₂O₃) or an oxygen-containing boron compound, which is boron oxide (B₂O₃) produced by thermal decomposition, and carbon (C) or pyrolytic carbon, which is carbon (C) produced by thermal decomposition, by the reaction represented by Equation (1) below:

B₂O₃ + 3C + N₂ → 2BN + 3CO (1)

This reaction is initiated at about 1400°C or more. The reaction temperature is preferably 1400°C to 1600°C, more preferably 1450°C to 1580°C. A lower reaction temperature leads to an increase in reaction time, while a higher reaction temperature makes it difficult to achieve a raw material boron nitride powder with uniform long diameter and thickness. The holding time is preferably 1 to 10 hours, more preferably 2 to 8 hours. A shorter holding time makes it difficult to achieve a raw material boron nitride powder with uniform long diameter and thickness.

After the reaction, the heating temperature is raised to 1620°C to 2100°C. With a rise in temperature, the crystallization of boron nitride particles proceeds, making the long diameter and the thickness larger, and the graphitization index lower. The maximum temperature is more preferably 1700°C to 2000°C, still more preferably 1750°C to 1900°C. A lower reaction temperature cannot achieve a raw material boron nitride powder with a graphitization index of 1.7 or less. A higher reaction temperature causes the particles to grow excessively to a size larger than that suitable for a raw material boron nitride powder, which results in a longer time required for wet-type jet milling. The maximum temperature is preferably held for 0.5 to 7 hours, more preferably 1 to 4 hours. A shorter holding time makes it difficult to achieve a raw material boron nitride powder with uniform long diameter and thickness, while a longer holding time leads to lower productivity.

Nitrogen gas, which is necessary as a nitrogen source for the reduction nitridation reaction, is preferably supplied to flow in a furnace. The flow path for nitrogen gas may be designed so that the nitrogen gas is allowed to flow in contact with the raw material mixture placed in a furnace (e.g., to flow above the layer of the raw material mixture). The amount of flowing nitrogen gas is preferably 0.1 to 20 L/minute, more preferably 0.2 to 15 L/minute, and still more preferably 0.3 to 10 L/minute, per kg of the raw material mixture. With a smaller amount of nitrogen gas, the reduction nitridation reaction is not completed, so that unreacted carbon unfavorably remains in the reaction product as a black foreign material. With an excessive amount of nitrogen gas, thermal efficiency is unfavorably reduced.

A container for the raw material mixture (R) may be prepared, such as a carbon container, a boron nitride-coated carbon container, or a boron nitride sintered body container, and the container filled with the raw material mixture may be placed in a furnace. The container preferably has a shape that does not prevent a contact between the raw material mixture (R) and flowing nitrogen gas.

The raw material mixture (R) preferably fills the container to a height of 1 to 20 cm. If the layer of the filling raw material mixture has a low height, productivity is reduced. If the layer of the filling raw material mixture has a too high height, the raw material mixture (R) at the bottom of the container remains unreacted, so that unreacted carbon unfavorably remains. The height of the layer of the filling raw material mixture (R) is preferably 2 to 15 cm, more preferably 3 to 10 cm.

### <Disintegration step, washing step, drying step and classification step>

The methods of disintegration, washing, drying and classification in the reduction nitridation method for producing the raw material boron nitride powder can be carried out by the same means under the same conditions as those described above for the melamine method.

### <Production of seed crystal (S)>

Next, the raw material boron nitride powder produced by the melamine method or the reduction nitridation method is cleaved, preferably followed by pulverization, thereby obtaining the seed crystal (S). It is important in the present invention to treat the raw material boron nitride powder with a wet-type jet mill.

With a wet-type jet mill, the particles are easily cleaved compared to a dry-type jet mill, resulting in a smaller (T90-T10)/T50 and a sharper thickness distribution. In addition, when the raw material boron nitride powder is in the form of a slurry with a density of 20 to 100 g/L, the particles can also be pulverized simultaneously during wet-type jet milling, resulting also in a smaller (L90-L10)/L50 and a sharper particle size distribution.

The wet-type jet milling is not particularly limited as long as it is capable of treating the slurry containing the raw material boron nitride powder and a solvent, but is preferably performed under the condition that the particles are efficiently cleaved, so that a (T90-T10)/T50 of 1.6 or less can easily be achieved. More specifically, it is preferable that: the solvent to be used is pure water, ethanol, isopropyl alcohol, N-methylpyrrolidone, N,N-dimethylformamide, polydimethylsiloxane, etc., or a compound with a surfactant added thereto; the slurry density is 20 to 100 g/L; the pressure is 100 to 300 MPa; and the slurry feed rate is 0.2 to 1.0 L/min. In Examples, Star Burst manufactured by Sugino Machine Limited was used with an oblique collision chamber nozzle. However, the present invention is not limited to this device, and any known wet-type jet milling device is applicable without limitation as long as it allows for pulverization in a like manner and achieves the effect of the present invention. A lower slurry density leads to less frequent collisions between particles, resulting in a decreased pulverization effect. To efficiently achieve a (L90-L10)/L50 of 2.0 or less, the slurry density is preferably 20 g/L or more, more preferably 40 g/L or more. If the slurry density is too high, the high viscosity may inhibit efficient wet-type jet milling. As such, the slurry density is preferably 100 g/L or less, more preferably 90 g/L or less. The number of passes of wet-type jet milling is not particularly limited, and jet milling may be performed until a desired specific surface area is obtained.

The wet-type jet milling is followed by filtration, drying and the like so that the solvent is removed from the slurry, thereby obtaining the seed crystal (S). If necessary, the filtrated slurry may be washed with pure water so that the solvent and surfactant used for the disintegration are removed, followed by drying. Further, prior to washing with pure water, the slurry may be washed with an organic solvent, acid or the like so that the solvent, surfactant, metallic impurities and the like used for the treatment are removed.

The wet-type jet milling may be followed by classification. Classification makes it easier to adjust the (L90-L10)/L50 and the (T90-T10)/T50 to 2.0 or less. The method of classification is not particularly limited. Considering that classification is successively performed after the pulverization by wet-type jet milling, wet classification is preferred. When classification can serve to remove coarse particles of the seed crystal (S), it becomes easier to obtain a boron nitride powder with small (L90-L10)/L50 and (T90-T10)/T50.

### <Production of hexagonal boron nitride powder>

In the present invention, a hexagonal boron nitride powder is produced by the reduction nitridation method, which involves heating the raw material mixture (S) containing an oxygen-containing boron compound, an oxygen-containing alkaline earth metal compound, a carbon source, and the seed crystal (S) in a nitrogen atmosphere.

### <Raw material mixture (S)>

For the oxygen-containing boron compound, the oxygen-containing alkaline earth metal compound, and the carbon source, which serve as reactive raw materials, the compounds as described above for the production of the raw material boron nitride powder by the reduction nitridation method can be used without limitation.

### <Composition ratio of raw material mixture (S) in reduction nitridation method>

In the reduction nitridation method, the reaction proceeds as represented by the Equation (1). The seed crystal (S) is not involved in the reduction nitridation (stoichiometric) reaction per se, but involved only in control of the long diameter and thickness of a boron nitride powder to be produced. As such, the atomic ratio (B/C ratio) of boron atoms (B) in the oxygen-containing boron compound to carbon atoms (C) in the carbon source, and the molar ratio (AO/B₂O₃ ratio) of the oxygen-containing alkaline earth metal compound (AO; A is an alkaline earth metal) to the oxygen-containing boron compound in terms of oxide can be the same as the quantitative ratios described above for the production of the raw material boron nitride powder.

The carbon source is blended so that the B/C ratio falls within the aforementioned range, resulting in a boron nitride powder with uniform long diameter and thickness. If the carbon source is used in a small amount, uniform long diameter and thickness are difficult to achieve. If the carbon source is used in a too large amount, unreacted carbon unfavorably remains in the boron nitride powder as a black foreign material. The oxygen-containing alkaline earth metal compound is blended so that the AO/B₂O₃ ratio falls within the aforementioned range, resulting in a boron nitride powder with intended long diameter and thickness, which contribute to good smoothness, reduced roughness, and excellent glossiness.

The seed crystal (S) is blended so that the atomic ratio (B^{S}/C ratio) of boron atoms (B^{S}) of the boron nitride powder in the seed crystal (S) to the carbon atoms (C) in the carbon source is preferably 0.01 to 13, more preferably 0.1 to 10, and still more preferably 0.5 to 8. The seed crystal (S) is blended so that the B^{S}/C ratio falls within this range, making it possible to obtain a boron nitride powder with uniform long diameter and thickness. If the seed crystal (S) is contained in a small amount, it does not have a positive effect as a seed crystal. If the seed crystal (S) is contained in a too large amount, productivity is disadvantageously reduced.

### <Heating step>

In the reduction nitridation method, the reaction proceeds as represented by the Equation (1). This reaction is initiated at about 1400°C or more. The reaction temperature is preferably 1400°C to 1600°C, more preferably 1450°C to 1580°C. A lower reaction temperature leads to an increase in reaction time, while a higher reaction temperature makes it difficult to achieve a boron nitride powder with uniform long diameter and thickness. The holding time is preferably 1 to 10 hours, more preferably 2 to 8 hours. A shorter holding time makes it difficult to achieve a boron nitride powder with uniform long diameter and thickness.

After the reaction, the heating temperature is raised to 1700°C to 2100°C. With a rise in temperature, the crystallization of boron nitride particles proceeds, making the long diameter and the thickness larger. The maximum temperature is more preferably 1730°C to 2000°C, still more preferably 1750°C to 1950°C. A lower reaction temperature does not allow the boron nitride powder to grow to a size suitable for a cosmetic, while a higher reaction temperature causes the boron nitride powder to grow excessively to a size larger than that suitable for a cosmetic. The maximum temperature is preferably held for 0.5 to 7 hours, more preferably 1 to 4 hours. A shorter holding time makes it difficult to achieve a boron nitride powder with uniform long diameter and thickness, while a longer holding time leads to lower productivity.

### <Nitrogen use conditions, reaction container, and raw material filling method>

In this reduction nitridation method, the nitrogen use conditions, the reaction container, and the raw material filling method can be the same as those described above for the production of the raw material boron nitride powder by the reduction nitridation method.

### <Disintegration step>

In order to reduce aggregated particles contained in the reaction product obtained by the reduction nitridation step, the reaction product is preferably disintegrated. The method of disintegration can be carried out by the same means under the same conditions as those described above for the production of the raw material boron nitride powder.

### <Classification step>

In order to reduce coarse particles contained in the reaction product obtained by the reduction nitridation step, the reaction product is preferably classified. The method of classification can be carried out by the same means under the same conditions as those described above for the production of the raw material boron nitride powder. The sieve opening is preferably in the above-described range for the reason that a hexagonal boron nitride powder with a lower MMD can easily be obtained.

### <Washing step>

The reaction product obtained by the reduction nitridation step contains impurities and the like, other than hexagonal boron nitride, such as unreacted raw materials (B₂O₃, CaO). Such impurities and the like are removed by washing the reaction product with acid, resulting in a high purity hexagonal boron nitride powder suitable for use in cosmetics. The method of washing can be carried out by the same means under the same conditions as those described above for the production of the raw material boron nitride powder.

The hexagonal boron nitride powder for use in cosmetics is desired to conform to the Japanese Standards of Quasi-drug Ingredients 2021, which specify a purity of 95% or more, a pH of 5.0 to 8.0, an elution amount of boron of 20 ppm or less, and the like. As such, the washing needs to be performed to meet these standards.

### <Drying step>

The method of drying can be carried out by the same means under the same conditions as those described above for the production of the raw material boron nitride powder. However, the boron nitride powder may be hydrolyzed during the drying step, possibly resulting in an elution amount of boron of more than 20 ppm. In view of this, vacuum drying, which is capable of suppressing hydrolysis, is preferred as the method of drying the boron nitride powder for the production of a hexagonal boron nitride powder.

### Examples

Hereinafter, the present invention will be described specifically by way of Examples; however, the present invention is not limited to the Examples. Further, not all the combinations of features described in the Examples are essential to solve the problems of the present invention.

In the following examples, a boron nitride powder before jet milling is referred to as a raw material boron nitride powder (or a raw material h-BN powder); a boron nitride powder after the jet milling is referred to as a seed crystal; a boron nitride powder obtained through a reduction nitridation step using the seed crystal is referred to as a nitride powder; and a boron nitride powder after washing and drying is referred to as a hexagonal boron nitride powder (or a h-BN powder). A powder for preparing the seed crystal is denoted by (S), as required.

A raw material h-BN powder was prepared by the melamine method or the reduction nitridation method.

### <Preparation of raw material h-BN powder (M1) by melamine method>

30 g of boron oxide (B₂O₃) as an oxygen-containing boron oxide and 50 g of melamine as a nitrogen-containing organic compound were mixed together with a ball mill to prepare a raw material mixture (S). The thus-prepared raw material mixture (S) had a B/N of 0.36.

The raw material mixture (S) was placed in a batch furnace in which nitrogen gas was flowing so that the mixture (S) was heated in a heating step at 15°C/minute to a temperature of 1800°C and kept heated at 1800°C for 6 hours, thereby preparing a nitride powder (M1). The thus-prepared nitride powder (M1) was disintegrated by a stone disintegrator and washed with acid in a 5% aqueous hydrochloric acid solution, followed by filtration, water washing, drying, and classification using a vibrating sieve with an opening of 45 µm, thereby obtaining a raw material h-BN powder (M1).

### <Preparation of raw material h-BN powder (M2) by melamine method>

A raw material h-BN powder (M2) was produced in the same manner as for the raw material h-BN powder M1, except that the heating temperature and the heating time were changed as shown in Table 1.

### <Preparation of raw material h-BN powder (M3) by melamine method>

A raw material h-BN powder (M3) was produced in the same manner as for the raw material h-BN powder M1, except that the heating temperature and the heating time were changed as shown in Table 1.

### <Preparation of raw material h-BN powder (M4) by melamine method>

A raw material h-BN powder (M4) was produced in the same manner as for the raw material h-BN powder M2, except for the absence of the classification using a vibrating sieve with an opening of 45 µm.

### <Preparation of raw material h-BN powder (R1) by reduction nitridation method>

70 g of boron oxide (B₂O₃) as an oxygen-containing boron oxide, 30 g of carbon black, and 10 g of calcium carbonate were mixed together with a ball mill. The mixture was placed in a graphite Tammann furnace so that the mixture was heated at 15°C/minute to a temperature of 1500°C and kept heated at 1500°C for 6 hours under a nitrogen gas atmosphere. Thereafter, the mixture was heated at 15°C/minute to a temperature of 1750°C and kept heated at 1750°C for 2 hours, thereby preparing a nitride powder (R1). The thus-prepared nitride powder (R1) was disintegrated by a stone disintegrator and washed with acid in a 5% aqueous hydrochloric acid solution, followed by filtration, water washing, drying, and classification using a vibrating sieve with an opening of 45 µm, thereby obtaining a raw material h-BN powder (R1).

### <Graphitization index>

The raw material h-BN powder was press-molded at 10 t into a h-BN pellet with approximately a diameter of 25 mm and a thickness of 3 mm as a test sample. With an X-ray diffractometer (D2 PHASER manufactured by Bruker), the X-ray diffraction spectrum of the test sample was measured and analyzed, based on which the graphitization index was calculated.

For the analysis of the X-ray diffraction spectrum, analysis software supplied with the X-ray diffractometer was used to obtain the peak areas of the planes (100), (101) and (102) of the h-BN. The peaks that appeared near 2θ = 41.5°, 44°, and 50° were regarded as corresponding to the planes (100), (101) and (102), respectively.

The graphitization index was calculated by the following formula, where Area (10X) is the peak area of the plane (10X). Graphitization index = (Area (100) + Area (101))/Area (102)

In a case where the crystallinity of the h-BN is too low, the peaks corresponding to the (100) and (101) may overlap, or the peak corresponding to the (102) may not appear. In the former case, an analysis may be made assuming that the peaks corresponding to the (100) and (101) are a single broad peak, and the peak area obtained by the analysis is substituted into the numerator of Formula (2), thereby calculating the graphitization index (which is usually more than 1.7). In the latter case, there is no calculating the graphitization index; thus, the graphitization index is regarded as more than 1.7.

### <Specific surface area>

The specific surface area was obtained by the BET method (the nitrogen adsorption single point method) using a specific surface area measuring device (Flow Sorb 2-2300 manufactured by SHIMADZU CORPORATION) .

### <Average long diameter, average thickness and aspect ratio>

The h-BN powder was treated with an ultrasonic homogenizer so that aggregated particles are disintegrated and monodispersed, followed by drying. The resultant powder was used as a measurement sample. More specifically, 2 g of the h-BN powder was mixed with 25 mL of ion exchange water to form a slurry, which was treated with ultrasound at an amplitude of 40% for 20 minutes using an ultrasonic homogenizer, SONIFIER (SFX250; chip: 20 kHz microchip 1/4 inch) manufactured by Branson Ultrasonics Corporation, followed by filtration and drying.

Then, 1 g of the h-BN powder was added to 9 g of epoxy resin (EA E-30CL manufactured by Henkel AG & Co. KGaA), followed by stirring and defoaming with MAZERUSTAR manufactured by Kurabo Industries Ltd. The resultant resin composition was poured into a mold 10 mm square and 1 mm thick and heated at a temperature of 70°C and cured, followed by cooling. The cured resin composition was demolded and cross-section milled at a single site on the side, followed by taking SEM images of a plurality of fields of view at 1000-fold magnification.

The thus-obtained SEM images were analyzed by an image analyzer (A-zou-kun manufactured by Asahi Kasei Engineering Corporation.), thereby measuring the major axes and thicknesses of the h-BN particles. The analysis mode of A-zou-kun was set for acicular materials. All the particles included in the SEM image of each of the fields of view were measured, except for particles, such as overlapping particles, that were difficult to precisely measure. This measurement was repeated for the plurality of fields of view. Finally, the measurement of the long diameter and thickness was performed for 1000 or more particles.

Next, the L50, T50 and the like in terms of area were analyzed by spreadsheet software (Microsoft Excel). Assuming that the h-BN particles were circular in planar shape and the long diameter of the particles was equivalent to the diameter of the circular shape, the area of (one of the two planar surfaces of) each of the h-BN particles was calculated. When the h-BN powder was arranged in ascending order of long diameter and thickness, the area-based cumulative 50% values were determined as the average long diameter (L50) and average thickness (T50) of the h-BN powder. The average long diameter divided by the average thickness was the aspect ratio (average long diameter (L50)/average thickness (T50)).

Further, the area-based cumulative 10% long diameter (L10), cumulative 50% long diameter (L50), and cumulative 90% long diameter (L90) of the h-BN powder as arranged in ascending order of long diameter were used to calculate (L90-L10)/L50. Similarly, the area-based cumulative 10% thickness (T10), cumulative 50% thickness (T50), and cumulative 90% thickness (T90) of the boron nitride particles, when the raw material h-BN powder was arranged in ascending order of thickness, were used to calculate (T90-T10)/T50.

### <MIU and MMD>

The MIU (dynamic friction coefficient) and the MMD (deviation of dynamic friction coefficient) are dimensionless numbers measured by a friction tester (manufactured by KATO TECH CO., LTD. under the trade name of KES-SE). More specifically, the h-BN powder was placed on artificial leather (manufactured by Idemitsu Technofine, Co., Ltd. under the trade name of SUPPLALE PBZ13001 BK) that simulated the skin, such that the powder was spread thinly in an amount of 0.5 mg/cm². Then, a (10-mm square silicon) sensor portion of the friction tester was applied onto the powder, thereby measuring the MIU and the MMD. The measurement conditions of the friction tester were set as follows: the sensitivity was H; the test table moving speed was 1 mm/second; and the static load was 25 gf. The measurement was performed once. The operation of preparing the specimen (by applying the raw material h-BN powder onto the artificial leather), followed by the measurement by the friction tester, was repeated five times. The average of the five measurements was determined as the MIU/MMD of the h-BN powder.

### <Specular reflection intensity>

Double-stick tape was applied to black matte flock paper and was entirely covered with the h-BN powder by a makeup brush, followed by blowing off excess powder with an air duster, thereby preparing a test sample. Light was allowed to be incident on the test sample at an angle of -60°, and the intensity of reflected light at an angle of -90° to +90° was measured by a goniophotometer (manufactured by Murakami Color Research Laboratory under the trade name of GP-200). The peak intensity of reflected light observed at an angle around +60° was regarded as the specular reflection intensity.

Table 1 shows the production method, production conditions, and measurement results for the raw material h-BN powders (M1)-(M4) and (R1). The raw material h-BN powder (M3) was so small in particle size that its long diameter distribution and thickness distribution were not able to be measured.

**[Table 1]**

| Name of raw material h-BN powder | | M1 | M2 | M3 | M4 | R1 |
|---|---|---|---|---|---|---|
| Production condition | Production method | Melamine | Melamine | Melamine | Melamine | Reduction nitridation |
| | Heating temperature | 1800°C | 1600°C | 1300°C | 1600°C | 1750°C |
| | Heating time | 6 hrs | 6 hrs | 6 hrs | 6 hrs | 2 hrs |
| | Classification | Performed | Performed | Performed | Not performed | Performed |
| | Graphitization index | 1.4 | 1.6 | 1.9 | 1.6 | 1.4 |
| | Specific surface area (m²/g) | 3 | 10 | 25 | 10 | 3 |
| | L50 (µm) | 7.9 | 3.4 | - | 3.4 | 6.2 |
| | T50 (µm) | 0.74 | 0.31 | - | 0.31 | 0.72 |
| Measurement result | Aspect ratio | 10.7 | 11.0 | - | 11.0 | 8.6 |
| | (L90-L10)/L50 | 2.3 | 2.4 | - | 2.4 | 2.6 |
| | (T90-T10)/T50 | 2.7 | 2.7 | - | 2.7 | 2.9 |
| | MIU | 0.50 | 0.53 | 0.55 | 0.56 | 0.52 |
| | MMD | 0.0052 | 0.0057 | 0.0058 | 0.0061 | 0.0054 |
| | Specular reflection intensity | 75 | 70 | 66 | 69 | 72 |

### <Example 1>

### <Preparation of seed crystal>

65 g of the raw material boron nitride powder (M1) obtained was mixed with 1 L of N,N-dimethylformamide and stirred sufficiently to form a slurry. The slurry was treated with a wet-type jet mill (Star Burst manufactured by Sugino Machine Limited) under the following conditions: the nozzle was an oblique collision chamber nozzle; the pressure was 200 MPa; the slurry feed rate was 0.5 L/min; and the number of passes was 5. The wet-type jet milled slurry was filtered by suction filtration, washed with pure water, and filtered by suction filtration, followed by vacuum drying at 200°C, thereby obtaining a seed crystal (S) (M1).

### <Raw material mixture>

50 g of boron oxide (B₂O₃) as an oxygen-containing boron compound, 8 g of calcium carbonate (CaCO₃) as an oxygen-containing alkaline earth metal compound, 24 g of carbon black (C) as a carbon source, and 40 g of the seed crystal (S) (M1) were mixed together with a ball mill to prepare a raw material mixture (S). The thus-prepared raw material mixture had a B/C (atomic ratio) of 0.72, a CaO/B₂O₃ (molar ratio) of 0.11, and a B^{S}/C (atomic ratio) of 0.81.

### <Reduction nitridation step>

The raw material mixture (S) prepared was placed in a batch furnace in which nitrogen gas was flowing so that the mixture (S) was heated at 1870°C for 2 hours, thereby producing a nitride powder (S).

### <Refinement step>

The thus-prepared nitride powder (S) was washed with acid in a 5% aqueous hydrochloric acid solution, followed by filtration, water washing, and vacuum drying at 200°C, thereby obtaining a h-BN powder (M1).

### <Classification step>

The thus-obtained h-BN powder was classified using a vibrating sieve with an opening of 45 µm.

### <Measurements>

The classified h-BN powder was measured for the items shown in Tables 3 to 5 in the same manner as for the raw material h-BN powder. The rate of coarse particles that were more than 2.5 times as large as the average long diameter (L50) was calculated by the following formula: Rate (area%) of coarse particles that were more than 2.5 times as large as average long diameter (L50) = 100 - (area-based cumulative value corresponding to value 2.5 times as large as L50)

### <Sensory evaluation>

The h-BN powder (M1) obtained was mixed with the following cosmetic components to prepare a cosmetic (powder foundation). The cosmetic prepared was evaluated for its smoothness, roughness, a moist texture, and glossiness by 20 research panelists specializing in cosmetic evaluation. The evaluation was made as follows: The cosmetic acceptable to less than 30% of the panelists was evaluated as ×; the cosmetic acceptable to not less than 30% and less than 60% of the panelists was evaluated as Δ; the cosmetic acceptable to not less than 60% and less than 80% of the panelists was evaluated as ∘; and the cosmetic acceptable to not less than 80% of the panelists was evaluated as **⊚.** Components of powder foundation and their blending amount (mass%)
· Hexagonal boron nitride powder (h-BN powder) 20.0
· Mica 15.0
Synthetic phlogopite 12.0
Ethylhexyl methoxycinnamate 8.0
· Vinyl dimethicone/methicone silsesquioxane crosspolymer 8.0
· Diphenyl dimethicone/vinyl diphenyl dimethicone/ silsesquioxane crosspolymer 8.0
· Nylon 12 3.0
· Silica 3.0
· Talc 3.0
· Acrylates crosspolymer 3.0
· Perfluorooctyl triethoxysilane 3.0
· Zinc oxide 3.0
· Polymethyl methacrylate polymer 3.0
· Silicone-treated colcothar (red iron oxide) 1.0
· Silicone-treated yellow iron oxide 0.6
· Silicone-treated black iron oxide 0.4
· Silicone-treated titanium oxide 6.0

### <Examples 2 to 11 and Comparative Examples 1 to 5>

In each of Examples 2 to 11 and Comparative Examples 1 to 5, a boron nitride powder was produced by changing any of the following conditions in Example 1: the type of the raw material h-BH power, the type of jet milling, the slurry density, the number of passes, the B/C atomic ratio and Cao/B₂O₃ molar ratio of the raw material mixture, the B^{S}/C atomic ratio, and the firing temperature. Further, the boron nitride powder produced was used to prepare a cosmetic in the same manner as in Example 1. The production conditions of the boron nitride and the evaluation results are shown in Tables 2 to 4. In Comparative Example 5, the boron nitride powder was produced without using a seed crystal.

**[Table 2]**

| | | Ex. 1 | Ex. 2 | Ex. 3 | Ex. 4 | Ex. 5 | Ex. 6 |
|---|---|---|---|---|---|---|---|
| Raw material h-BN powder | | M1 | M1 | M1 | M1 | M1 | M1 |
| Jet milling | Wet/dry type | Wet | Wet | Wet | Wet | Wet | Wet |
| | Slurry density (g/L) | 65 | 65 | 90 | 30 | 65 | 65 |
| | Number of passes | 5 | 12 | 5 | 5 | 5 | 5 |
| Seed crystal (S) | Specific surface area (m²/g) | 12 | 22 | 15 | 18 | 12 | 12 |
| | Seed crystal (S) (g) | 40 | 40 | 40 | 40 | 40 | 40 |
| | B₂O₃ (g) | 50 | 50 | 50 | 50 | 60 | 50 |
| | C (g) | 24 | 23 | 23 | 23 | 16 | 9 |
| | CaCÖ₃ (g) | 8 | 5 | 5 | 5 | 25 | 60 |
| Raw material mixture and production condition | B/C (atomic ratio) | 0.72 | 0.75 | 0.75 | 0.75 | 1.29 | 1.92 |
| | CaO/B₂O₃ (molar ratio) | 0.11 | 0.07 | 0.07 | 0.07 | 0.29 | 0.83 |
| | B^{S}/C (atomic ratio) | 0.81 | 0.84 | 0.84 | 0.84 | 1.21 | 2.15 |
| | Firing temperature (°C) | 1870 | 1800 | 1800 | 1800 | 1800 | 1800 |
| | Holding time | 2 hrs | 2 hrs | 2 hrs | 2 hrs | 2 hrs | 2 hrs |
| | Sieve opening (µm) | 45 | 45 | 45 | 45 | 45 | 45 |
| | L50 (µm) | 15.4 | 7.8 | 8.2 | 9.0 | 8.4 | 11.3 |
| | T50 (µm) | 1.11 | 0.41 | 0.64 | 0.40 | 0.63 | 0.83 |
| | Aspect ratio | 13.9 | 19.0 | 12.8 | 22.5 | 13.3 | 13.6 |
| | (L90-L10)/L50 | 1.6 | 1.1 | 1.3 | 1.8 | 1.3 | 1.3 |
| | (T90-T10)/T50 | 1.7 | 1.3 | 1.8 | 1.3 | 1.7 | 1.6 |
| h-BN powder | Rate of coarse particles more than 2.5 times as large as L50 (area%) | 5 | 1 | 2 | 6 | 4 | 2 |
| | MIU | 0.37 | 0.33 | 0.38 | 0.34 | 0.38 | 0.36 |
| | MMD | 0.0027 | 0.0025 | 0.0030 | 0.0026 | 0.0026 | 0.0026 |
| | Specular reflection intensity | 90 | 92 | 84 | 93 | 86 | 92 |
| Foundation | Smoothness | ⊚ | ⊚ | ⊚ | ⊚ | ⊚ | ⊚ |
| | Roughness | ⊚ | ⊚ | ⊚ | ⊚ | ⊚ | ⊚ |
| | Moist texture | ⊚ | ⊚ | ⊚ | ⊚ | ⊚ | ⊚ |
| | Glossiness | ⊚ | ⊚ | ○ | ⊚ | ○ | ⊚ |

| | | | | | | | |
|---|---|---|---|---|---|---|---|
| Ex.: Example | | | | | | | |

**[Table 3]**

| | | Ex. 7 | Ex. 8 | Ex. 9 | Ex. 10 | Ex. 11 |
|---|---|---|---|---|---|---|
| Raw material h-BN powder | | M1 | M1 | M2 | R1 | M4 |
| Jet milling | Wet/dry type | Wet | Wet | Wet | Wet | Wet |
| | Slurry density (g/L) | 65 | 65 | 65 | 65 | 65 |
| | Number of passes | 5 | 5 | 5 | 10 | 8 |
| Seed crystal (S) | Specific surface area (m²/g) | 12 | 12 | 19 | 14 | 19 |
| | Seed crystal (S) (g) | 300 | 40 | 40 | 40 | 40 |
| | B₂O₃ (g) | 70 | 50 | 50 | 50 | 50 |
| | C (g) | 23 | 23 | 23 | 23 | 23 |
| | CaCÖ₃ (g) | 15 | 5 | 5 | 5 | 5 |
| Raw material mixture and production condition | B/C (atomic ratio) | 1.05 | 0.75 | 0.75 | 0.75 | 0.75 |
| | CaO/B₂O₃ (molar ratio) | 0.15 | 0.07 | 0.07 | 0.07 | 0.07 |
| | B^{S}/C (atomic ratio) | 6.31 | 0.84 | 0.84 | 0.84 | 0.84 |
| | Firing temperature (°C) | 1800 | 1760 | 1800 | 1800 | 1800 |
| | Holding time | 2 hrs | 2 hrs | 2 hrs | 2 hrs | 2 hrs |
| | Sieve opening (µm) | 45 | 45 | 45 | 45 | 45 |
| | L50 (µm) | 7.5 | 6.5 | 7.9 | 8.6 | 8.1 |
| | T50 (µm) | 0.52 | 0.44 | 0.56 | 0.80 | 0.60 |
| | Aspect ratio | 14.4 | 14.8 | 14.1 | 10.8 | 13.5 |
| | (L90-L10)/L50 | 1.2 | 1.2 | 1.4 | 1.8 | 1.6 |
| h-BN powder | (T90-T10)/T50 | 1.4 | 1.5 | 1.8 | 1.9 | 1.9 |
| | Rate of coarse particles more than 2.5 times as large as L50 (area%) | 1 | 2 | 2 | 6 | 3 |
| | MIU | 0.37 | 0.35 | 0.37 | 0.39 | 0.39 |
| | MMD | 0.0027 | 0.0026 | 0.0026 | 0.0038 | 0.0033 |
| | Specular reflection intensity | 92 | 90 | 84 | 82 | 83 |
| Foundation | Smoothness | ⊚ | ⊚ | ⊚ | ⊚ | ⊚ |
| | Roughness | ⊚ | ⊚ | ⊚ | ⊚ | ⊚ |
| | Moist texture | ⊚ | ⊚ | ⊚ | ○ | ⊚ |
| | Glossiness | ⊚ | ⊚ | ○ | ○ | ○ |

| | | | | | | |
|---|---|---|---|---|---|---|
| Ex.: Example | | | | | | |

**[Table 4]**

| | | Compar. Ex. 1 | Compar. Ex. 2 | Compar. Ex. 3 | Compar. Ex. 4 | Compar. Ex. 5 |
|---|---|---|---|---|---|---|
| Raw material h-BN powder | | M1 | M1 | M2 | M3 | None |
| Jet milling | Wet/dry type | Wet | Dry | Not pulverized | Wet | - |
| | Slurry density (g/L) | 65 | - | - | 65 | - |
| | Number of passes | 3 | 1 | - | 12 | - |
| Seed crystal (S) | Specific surface area (m²/g) | 8 | 12 | 10 | 36 | - |
| | Seed crystal (S) (g) | 40 | 40 | 40 | 40 | - |
| | B₂O₃ (g) | 50 | 50 | 50 | 50 | 50 |
| | (g) | 23 | 23 | 23 | 23 | 23 |
| | CaCÖ₃ (g) | 5 | 5 | 5 | 5 | 5 |
| | B/C (atomic ratio) | 0.75 | 0.75 | 0.75 | 0.75 | 0.75 |
| Raw material mixture and production condition | CaO/B₂O₃ (molar ratio) | 0.07 | 0.07 | 0.07 | 0.07 | 0.07 |
| | B^{S}/C (atomic ratio) | 0.84 | 0.84 | 0.84 | 0.84 | |
| | Firing temperature (°C) | 1800 | 1800 | 1800 | 1800 | 1800 |
| | Holding time | 2 hrs | 2 hrs | 2 hrs | 2 hrs | 2 hrs |
| | Sieve opening (µm) | 45 | 45 | 45 | 45 | 45 |
| | L50 (µm) | 8.0 | 8.4 | 8.8 | 8.3 | 8.4 |
| | T50 (µm) | 0.67 | 0.96 | 0.95 | 0.88 | 1.02 |
| | Aspect ratio | 11.9 | 8.8 | 9.3 | 9.4 | 8.2 |
| | (L90-L10)/L50 | 2.0 | 1.2 | 2.1 | 2.1 | 2.5 |
| | (T90-T10)/T50 | 2.1 | 2.2 | 2.3 | 2.4 | 2.8 |
| h-BN powder | Rate of coarse particles more than 2.5 times as large as L50 (area%) | 7 | 1 | 10 | 10 | 12 |
| | MIU | 0.44 | 0.38 | 0.45 | 0.45 | 0.51 |
| | MMD | 0.0040 | 0.0031 | 0.0039 | 0.0037 | 0.0052 |
| | Specular reflection intensity | 78 | 78 | 77 | 77 | 74 |
| Foundation | Smoothness | Δ | ○ | ○ | Δ | × |
| | Roughness | ⊚ | ⊚ | ○ | ○ | Δ |
| | Moist texture | Δ | ○ | × | × | × |
| | Glossiness | × | Δ | × | × | × |

| | | | | | | |
|---|---|---|---|---|---|---|
| Compar. Ex.: Comparative Example | | | | | | |

As shown in Tables 2 to 4, the cosmetics using the hexagonal boron nitride powder of the present invention received high evaluation marks for smoothness, roughness, a moist texture, and glossiness.

Comparative Example 1 used a seed crystal with a small specific surface area, resulting in extremely poor glossiness.

In Comparative Example 2, the raw material boron nitride was subjected to dry-type jet milling, resulting in poor glossiness.

In Comparative Example 3, the raw material boron nitride was not pulverized by a jet mill or the like, resulting in extremely poor moist texture and glossiness.

Comparative Example 4 used the raw material h-BN powder (M3), which was produced by the melamine method at a low heating temperature. The resultant h-BN powder was not satisfactory in (L90-L10)/L50, (T90-T10)/T50, and average aspect ratio, resulting in extremely poor moist texture and glossiness.

Comparative Example 5 used a boron nitride powder obtained by the reduction nitridation method without using a seed crystal, resulting in unsatisfactory powder foundation that was inferior in all the physical properties.

### Explanations of Letters or Numerals:

- 1:: Hexagonal boron nitride powder
- 3:: Incident light
- 5:: Reflected light

## Claims

1. A hexagonal boron nitride powder, having:
an average long diameter (L50) of 3 to 20 µm, an average thickness (T50) of 0.3 to 2 µm, a (L90-L10)/L50 of 2.0 or less, a (T90-T10)/T50 of 2.0 or less, and an average aspect ratio of 10 to 30;
a dynamic friction coefficient (MIU) of 0.50 or less, and a deviation of the dynamic friction coefficient (MMD) of 0.0050 or less; and
a specular reflection intensity of 80 or more as measured by a goniophotometer under a condition of an angle of incidence of 60°.

2. The hexagonal boron nitride powder according to claim 1, wherein the (L90-L10)/L50 is 1.8 or less.

3. The hexagonal boron nitride powder according to claim 1, for use in a cosmetic.

4. A cosmetic including the hexagonal boron nitride powder according to claim 1.

5. A method for producing a hexagonal boron nitride powder, including reducing and nitriding a raw material mixture by heating in a nitrogen atmosphere, wherein
the raw material mixture contains an oxygen-containing boron compound, an oxygen-containing alkaline earth metal compound, a carbon source, and a seed crystal, and
the seed crystal is a boron nitride powder with a specific surface area of 10 m²/g or more obtained by subjecting a raw material boron nitride powder with a graphitization index of 1.7 or less to wet-type jet milling.

6. The method for producing a hexagonal boron nitride powder according to claim 5, wherein the raw material boron nitride powder to be subjected to wet-type jet milling is in the form of a slurry with a density of 20 to 100 g/L.

7. The method for producing a hexagonal boron nitride powder according to claim 5, wherein the raw material mixture has:
an atomic ratio (B/C ratio) of boron atoms (B) in the oxygen-containing boron compound to carbon atoms (C) in the carbon source of 0.70 to 2.00;
a molar ratio (AO/B₂O₃ ratio) of the oxygen-containing alkaline earth metal compound (AO; A is an alkaline earth metal) to the oxygen-containing boron compound (B₂O₃) of 0.01 to 1.0 in terms of oxide; and
an atomic ratio (B^{S}/C ratio) of boron atoms (B^{S}) in the boron nitride powder as the seed crystal to the carbon atoms (C) in the carbon source of 0.01 to 13.

8. The method for producing a hexagonal boron nitride powder according to claim 5, wherein the raw material mixture is reduced and nitrided by heating at a temperature of 1700°C to 2100°C.
